# EUROPEAN PATENT APPLICATION

(11) **EP 2 363 115 A2**
(43) Date of publication of application: **07.09.2011**
(21) Application number: 11167517.9
(22) Date of filing: 22.06.2006
(51) Int. Cl.: A61K 9/16, A61K 47/14, A61K 47/10, A61K 47/44, A61K 47/26, A61K 47/32, A61K 47/12, A61K 47/08

(54) **Complexation of metal ions with polypeptides**

(30) Priority: 21.06.2006 US 471984; 23.06.2005 US 693173 P
(62) Divisional of application: 06785698.9
(71) Applicant: ALZA Corporation, Mountain View, CA 94039 (US)
(72) Inventor: Rohloff, Catherine Manya, Los Altos, CA CA 94024 (US); Chen, Guohua, Sunnyvale, CA CA 94086 (US); Ding, Zhongli, Sunnyvale, CA CA 94087 (US); Berry, Stephen Andrew, Longview, WA WA 98632 (US); Narayanan, Latha Pisharody, Milpitas, CA CA 95035 (US); Desjardin, Michael A, Sunnyvale, CA CA 94087 (US)
(74) Representative: Woods, Geoffrey Corlett

(57) **Abstract**

Formulations and methods are provided for improving the stability upon exposure to aqueous media of polypeptides present in non-aqueous suspension vehicles. In particular aspects of the invention, formulations are provided that comprise a complex of a metal ion and a polypeptide suspended in a non-aqueous, biocompatible suspension vehicle. Aggregation of individual polypeptide molecules is reduced when aqueous media is introduced to such formulations, serving to stabilize the polypeptide.

## Description

### CROSS REFERENCE TO RELATED APPLICATIONS

This application claims the benefit of United States application serial number 60/693,173, filed June 23, 2005, which is incorporated herein in its entirety.

### FIELD OF THE INVENTION

Particular aspects of the present invention relate to formulations and methods for improving the stability upon exposure to aqueous media of polypeptides suspended in non-aqueous vehicles.

### BACKGROUND OF THE INVENTION

Proteins have utility as pharmaceuticals for the prevention, treatment, and diagnosis of disease. Proteins are naturally active in aqueous environments, and preferred protein formulations are thus aqueous solutions. Proteins are typically only marginally stable in aqueous solutions, however, and aqueous pharmaceutical preparations exhibit short shelf-lives at ambient or physiological temperatures and thus often require refrigeration. In addition, the solubility of many proteins in aqueous solutions is limited, and proteins that are soluble at high concentrations in aqueous solutions are prone to aggregation and precipitation. Moreover, water acts as a plasticizer and facilitates the unfolding and irreversible molecular aggregation of protein molecules. Non-aqueous or substantially non-aqueous protein formulations are thus generally required to ensure protein stability over time at ambient or physiological temperatures.

One means to prepare non-aqueous protein formulations is to reduce aqueous protein formulations to dry powders. Protein formulations can be dried using various techniques, including freeze-drying, spray-drying, lyophilization, and dessication. Dry powder protein formulations exhibit significantly increased stability over time at ambient or even physiological temperatures. But where a flowable protein formulation is required, such as for parenteral injection or for use in implantable delivery devices, dry powder protein formulations are of limited use.

Dry protein powders can be suspended in non-aqueous, flowable vehicles, however, and such suspensions are stable at ambient or even physiologic temperatures over prolonged periods of time. It has been found that proteins suspended within non-aqueous vehicles can precipitate when the proteins are exposed to aqueous media, however. It is believed that when proteins contained within the non-aqueous suspension vehicles are exposed to aqueous environmental fluid the proteins may denature and subsequently aggregate, resulting in precipitation of the proteins. A need therefore exists in the art for protein formulations and methods that improve the stability of proteins suspended in non-aqueous vehicles upon exposure of the proteins to aqueous media.

### SUMMARY OF THE INVENTION

In certain aspects, the present invention relates to formulations comprising a complex of a metal ion and a polypeptide suspended in a non-aqueous, biocompatible suspension vehicle wherein, upon exposure of the formulation to aqueous media, the polypeptide does not aggregate to a substantial degree. In other embodiments, the invention is directed to methods for improving the stability upon exposure to aqueous media of a polypeptide suspended in a non-aqueous biocompatible suspension vehicle comprising forming a complex of the polypeptide and a metal ion.

### BRIEF DESCRIPTION OF THE DRAWINGS

Figure 1 is a graph illustrating the stability of human growth hormone and human growth hormone/zinc ion complex.

Figure 2 is a graph illustrating the fraction of human growth hormone and human growth hormone/zinc ion complex that is soluble in phosphate-buffered saline.

Figure 3 is a graph depicting the results of microcalorimetry experiments performed on mixtures of zinc and omega-IFN.

### DETAILED DESCRIPTIONS ILLUSTRATIVE EMBODIMENTS

Certain embodiments of the present invention relate to formulations that improve the stability upon exposure to aqueous media of polypeptides suspended in non-aqueous, biocompatible suspension vehicles. The formulations comprise a complex of a polypeptide and a metal ion suspended in a non-aqueous suspension. Upon exposure to aqueous media, the polypeptide remains stable and does not aggregate to a substantial degree. Other aspects of the invention relate to methods for improving the stability upon exposure to aqueous media of polypeptides suspended in non-aqueous, biocompatible suspension vehicles that comprise forming a complex of a polypeptide and a metal ion. The polypeptide/metal ion complex remains stable upon exposure to aqueous media, and the polypeptide does not aggregate to a substantial degree.

As used herein, the term "complex" refers to a composition that comprises a polypeptide coordinated to at least one metal ion. By "coordinated" it is meant that one atom of the polypeptide forms a bond with the metal ion, where the polypeptide atom is a Lewis base donor atom, and the metal ion is a Lewis acid acceptor atom.

As used herein, the term "suspension" refers to a composition that is at least bi-phasic in that it contains a continuous phase and at least one discontinuous phase. The term "suspended" refers to the state of the substance that is in the discontinuous phase of a suspension.

As used herein, the term "suspension vehicle" refers to the continuous phase of a suspension. In certain embodiments of the invention a polypeptide/metal ion complex is suspended in a suspension vehicle. A polypeptide/metal ion complex will generally remain in its original physical form throughout the lifespan of a dosage form containing a polypeptide/metal ion complex suspended in a suspension vehicle. For example, polypeptide/metal ion complexes that are solid particulates will generally remain particles throughout the lifespan of a dosage form containing the particulate polypeptide/metal ion complexes suspended in a suspension vehicle.

As used herein, the term "non-aqueous" refers to a substance that is substantially free of water. Non-aqueous liquids preferably comprise less than about 5% water, more preferably less than about 2% water, and most preferably less than about 1% water, by weight.

As used herein, the term "aqueous media" refers to substances that contain some water and may also contain one or more other substances, such as, for example, salts, that form multi-component solutions with water. Aqueous media preferably comprise at least 50% water, more preferably at least 60% water, and most preferably at least 75% water, by weight.

As used herein, the phrase "insoluble in aqueous media," refers to a substance that cannot be dissolved to a substantial degree in aqueous media. A substance is "dissolved to a substantial degree" in aqueous media if more than a trace or trivial amount of the substance is dissolved in aqueous media.

As used herein, the term "aggregate" refers to the process by which individual polypeptide molecules associate, gather, or join together. Polypeptides "do not aggregate to a substantial degree" if no more than a trace or trivial amount of polypeptides aggregate.

As used herein, the term "biocompatible" refers to substances that are physiologically acceptable to a living tissue and organism. Biocompatible substances will not cause a significant adverse reaction or response when introduced into a majority of patients.

As used herein, the phrase "exposure to aqueous media" refers to the introduction of a formulation comprising a complex of a metal ion and a polypeptide present in a non-aqueous vehicle to any measurable amount of an aqueous media.

As used herein, the phrase "improving the stability upon exposure to aqueous media" refers to any measurable reduction in the aggregation of protein molecules that occurs upon exposure to aqueous media of a non-aqueous formulation in which the protein molecules are present.

As used herein, the term "polypeptide" refers to peptides, proteins, polymers of amino acids, hormones, viruses, and antibodies that are naturally derived, synthetically produced, or recombinantly produced. Polypeptides also include lipoproteins and post translationally modified proteins, such as, for example, glycosylated proteins, as well as proteins or protein substances that have D-amino acids, modified, derivatized, or non-naturally occurring amino acids in the D- or L- configuration and/or peptomimetic units as part of their structure.

Polypeptides are typically unstable in hydrophobic/hydrophilic interfaces. Specifically, polypeptides formulated in non-aqueous vehicles are unstable when exposed to aqueous media. Polypeptides to be delivered by injection or from implantable delivery devices are typically formulated as particles suspended in non-aqueous vehicles to ensure the stability of the polypeptides over extended periods of time at physiological temperatures. Instability occurs, however, when the polypeptides enter the aqueous environment of use. It is believed that when polypeptides suspended in non-aqueous vehicles encounter aqueous fluid the polypeptides may unfold and subsequently aggregate, resulting in precipitation of the polypeptides. It is further believed that if polypeptides suspended in non-aqueous vehicles are locked into their native conformation, potential denaturation of the polypeptides is greatly reduced upon exposure to aqueous media, thereby preventing aggregation and concomitant precipitation of the polypeptides.

Accordingly, certain embodiments of the invention are directed to formulating polypeptides so that the polypeptides are locked into their native conformation by chelating the polypeptides with metal ions. It is believed that when inter-molecular chelation occurs, three-dimensional networks form, which lock the polypeptides into their native conformation. Upon exposure to aqueous media, the metal ion/polypeptide complexes are not susceptible to denaturation, preventing aggregation of the polypeptides. Accordingly, when metal ion/polypeptide complexes present in non-aqueous vehicles are delivered by injection or from implantable delivery devices, aggregation does not occur during the transition of the polypeptides the aqueous environment of use. Particular aspects of the invention thus relate to polypeptide/metal ion complexes stably formulated as suspensions in non-aqueous, viscous vehicles for delivery by injection or for sustained delivery at physiological temperatures from implantable delivery devices.

Certain aspects of the present invention relate to formulations comprising polypeptide/metal ion complexes that are suspended in non-aqueous, biocompatible suspension vehicles. The complexed polypeptides do not aggregate to a substantial degree upon exposure of the formulations to aqueous media. In preferred embodiments of the invention, the polypeptide/metal ion complexes are insoluble in aqueous media. In other embodiments of the invention, the polypeptide/metal ion complexes are soluble in aqueous media.

Further embodiments of the invention relate to methods for improving the stability upon exposure to aqueous media of polypeptides suspended in non-aqueous, biocompatible suspension vehicles that comprise forming complexes of the polypeptides and metal ions.

Polypeptides that can be completed with metal ions include, but are not limited to, peptides, proteins, polymers of amino acids, hormones, viruses, antibodies, etc. that are naturally derived and synthetically or recombinantly produced. The polypeptides also include lipoproteins and post translationally modified forms, e.g., glycosylated proteins, as well as proteins or protein substances that have D-amino acids, modified, derivatized or non-naturally occurring amino acids in the D- or L- configuration and/or peptidomimetic units as part of their structure. Preferably, the polypeptides are bone morphogenic proteins, insulin, colchicine, glucagon, thyroid stimulating hormone, parathyroid and pituitary hormones, calcitonin, renin, prolactin, corticotrophin, thyrotropic hormone, follicle stimulating hormone, chorionic gonadotropin, gonadotropin releasing hormone, bovine somatotropin, porcine somatotropin, oxytocin, vasopressin, GRF, somatostatin, lypressin, pancreozymin, luteinizing hormone, LHRH, LHRH agonists and antagonists, leuprolide, interferons of mammalian origin such as interferon alpha-2a, interferon alpha-2b, interferon tau, interferon omega, and consensus interferon, interleukins, growth factors such as epidermal growth factors (EGF), platelet-derived growth factors (PDGF), fibroblast growth factors (FGF), transforming growth factors-α (TGF-α), transforming growth factors-β (TGF-β), erythropoietin (EPO), insulin-like growth factor-I (IGF-I), insulin-like growth factor-II (IGF-II), interleukin-1, interleukin-2, interleukin-6, interleukin-8, tumor necrosis factor-α (TNF-α), tumor necrosis factor-β (TNF-β), Interferon-β (INF-β), Interferon-γ (INF-γ), colony stimulating factors (CGF), vascular cell growth factor (VEGF), thrombopoietin(TPO), stromal cell-derived factors (SDF), placenta growth factor (PIGF), hepatocyte growth factor (HGF), granulocyte macrophage colony stimulating factor (GM-CSF), glial-derived neurotrop in factor (GDNF), granulocyte colony stimulating factor (G-CSF), ciliary neurotropic factor (CNTF), bone morphogeneic proteins (BMP), coagulation factors, or human pancreas hormone releasing factor.

It may be desirable to combine different polypeptides in particular aspects of the invention. As such, any of the foregoing examples of polypeptides can be completed to a metal ion either alone, or in combination with other polypeptides.

Preferred metal ions that can be complexed to polypeptides are multivalent metal ions and include, for example, zinc, magnesium, calcium, nickel, and copper. Zinc is a particularly preferred metal ion.

In preferred aspects of the invention, the molar ratio of the metal ion to the polypeptide in the metal ion/polypeptide complex is from about 1:1 to about 100:1. In more preferred embodiments of the invention, the molar ratio of the metal ion to the polypeptide is from about 10:1 1 to about 50:1. In particularly preferred embodiments of the invention, the molar ratio of the metal ion to the polypeptide is from about 15:1 to about 30:1.

In particular embodiments of the invention, the metal ion/polypeptide complexes are dried to form particles. The diameter of the particles is preferably between about 0.3 and about 50 microns, and more preferably, from about 1 to about 10 microns. In preferred embodiments of the invention, particles of the polypeptide/metal ion complexes are prepared by milling, sieving, spray drying, or supercritical fluid extraction.

According to certain embodiments of the invention, the polypeptide/metal ion complexes are stably suspended in non-aqueous vehicles. In general, the suspension vehicles are single-phase, viscous, flowable compositions that are substantially formed of non-aqueous, biodegradable, biocompatible materials. The polypeptide/metal ion complexes preferably exhibit little or no solubility in the suspension vehicles.

Polypeptide/metal ion complex suspensions according to certain embodiments of the present invention can be prepared by dispersing a desired polypeptide/metal ion complex within a suspension vehicle using any suitable means or method known in the art. The polypeptide/metal ion complex can be provided in any desirable form that allows dispersion of the beneficial agent within a suspension vehicle. Before dispersion within a suspension vehicle, the polypeptide/metal ion complex is preferably provided in a stabilized dry powder form. The polypeptide/metal ion complexes included in suspensions according certain embodiments of the present invention are generally degradable in water but stable as dry powders at ambient and physiological temperatures. Preferably, suspensions remain substantially homogenous for about 3 months, even more preferably for about 6 months, and yet even more preferably, for about 1 year. In addition, the polypeptide/metal ion complex remains physically and chemically stable in the suspension vehicle for about 3 months, even more preferably for about 6 months, and yet even more preferably, for about 1 year.

The non-aqueous, biocompatible vehicles used to suspend the polypeptide/metal ion complexes according to certain aspects of the invention optionally comprise at least one of a polymer, a solvent, or a surfactant. In preferred embodiments of the invention, the suspension vehicles comprise at least one of a polymer or a solvent. In further preferred embodiments of the invention, the suspension vehicles comprise both a polymer and a solvent and optionally comprise a surfactant.

Polymers that can be used to prepare the non-aqueous suspension vehicles include, for example, polyesters such as polylactic acid (PLA) (having an inherent viscosity in the range of about 0.5 to 2.0 i.v.) and polylacticpolyglycolic acid (PLGA) (having an inherent viscosity in the range of about 0.5 to 2.0 i.v.), pyrrolidones such as polyvinylpyrrolidone (having a molecular weight range of about 2,000 to 1,000,000), esters or ethers of unsaturated alcohols such as vinyl acetate, and polyoxyethylenepolyoxypropylene block copolymers (exhibiting a high viscosity at 37°C) such as Pluronic 105. Preferred polymers include polyvinylpyrrolidone.

Solvents that can be used to prepare the suspension vehicles include, for example, carboxylic acid esters such as lauryl lactate, polyhydric alcohols such as glycerin, polymers of polyhydric alcohols such as polyethylene glycol (having a molecular weight of about 200 to 600), fatty acids such as oleic acid and octanoic acid, oils such as castor oil, propylene carbonate, benzyl benzoate, lauryl alcohol, benzyl alcohol, or esters of polyhydric alcohols such as triacetin acetate. Preferred solvents include lauryl lactate.

The suspension vehicles can contain surfactants, including, for example, esters of polyhydric alcohols such as glycerol monolaurate, ethoxylated castor oil, polysorbates, esters or ethers of saturated alcohols such as myristyl lactate (Ceraphyl 50), and polyoxyethylenepolyoxypropylene block copolymers such as Pluronic. Preferred surfactants include gylcerol monolaurate and polysorbates.

The suspension vehicles can also contain excipients such as, for example, antioxidants, stabilizers, and viscosity modifiers. Regardless of the type of excipient used, excipient materials included in the suspension vehicles preferably account for no more than about 25 wt % of the suspension vehicle, and in preferred embodiments where excipients are used, the suspension vehicle includes no more than about 15 wt %, 10 wt % or 5 wt % excipient material.

The non-aqueous vehicles can be loaded with varying amounts of the polypeptide/metal ion complex to provide formulations that allow dosing of the polypeptide at a desired rate over a chosen period of time. Polypeptide/metal ion complex formulations according to preferred embodiments of the present invention include about 0.1 wt % to about 15 wt % polypeptide/metal ion complex, depending on the potency of the polypeptide, and more preferably, from about 0.4 wt % to about 5 wt %. If the polypeptide/metal ion complex is dispersed within a suspension vehicle as a particulate material, the polypeptide/metal ion complex particles, which may contain varying amounts of the polypeptide/metal ion complex and one or more excipients, preferably account for no more than about 25 wt % of the polypeptide/metal ion complex suspension.

Suspending vehicles and polypeptide/metal ion complex suspensions can be prepared for use in all types of dosage forms, e.g., oral suspensions, ophthalmologic suspensions, implant suspensions, injection suspensions, and infusion suspensions. A preferred dosage form is an implantable osmotic dosage form. Osmotically-driven, also referred to as pump-driven, devices include those described in U.S. Patent Nos. 5,985,305; 6,113,938; 6,132,420, 6,156,331; 6,395,292, each of which is incorporated herein by reference in its entirety.

The polypeptide/metal ion complex suspensions according to certain embodiments of the present invention can be formulated to allow dispensing from an implantable delivery device at a desired flow rate. In particular, a polypeptide/metal ion complex suspension can be formulated for delivery at flow rates of up to about 5 µl/day, depending on the polypeptide/metal ion complex to be delivered and the implantable device used to deliver the polypeptide/metal ion complex suspension. Where the polypeptide/metal ion complex is delivered from an osmotically driven implantable device designed to provide low flow rates, the polypeptide/metal ion complex suspension is preferably formulated for delivery of between about 0.5 and 5 µl/day, with flow rates of about 1.5 µl/day and 1.0 µl/day being particularly preferred.

It is preferable that the suspending vehicle is physiologically acceptable for a desired route of administration, for example, there are no adverse biological responses by the recipient of the suspension upon administration. In some embodiments of the present invention, it is preferable that the components are suitable for parenteral routes of administration, including but not limited to injection, infusion, or implantation.

The following examples are illustrative of certain embodiments of the invention and should not be considered to limit the scope of the invention.

### Example 1: Preparation of non-aqueous single phase viscous vehicles

Benzyl benzoate (BB) (22.81 g) and benzyl alcohol (BA) (obtained from J.T Baker) (2.55 g) were mixed in a beaker in a nitrogen filled glove box. Polyvinylpyrrolidone C30 (BASF, Mount Olive, N.J.) (9.96 g) was added to the mixture of BB/BA (9.58 g) in a glass vessel in a nitrogen filled glove box. The mixture was manually stirred with a spatula to wet the polymer powder completely. The mixture was further stirred at 65°C with a spatula attached to an overhead mixer until a single phase was achieved.

Additional, non-aqueous single phase viscous vehicles, the compositions of which are shown in Table 1 below, were prepared according to the procedures described above.

**Table 1**

| **Formulation** | **Polymer** | **Surfactant** | **Solvent** | **Ratio** | **Viscosity (Poise)** |
|---|---|---|---|---|---|
| 1 | PVP | GML | LL | 53:5:42 | 25,000 |
| 2 | PVP | GML | LL | 55:10:35 | 50,000 |
| 3 | PVP | GML | LL | 50:15:35 | 7,000 |
| 4 | PVP | — | LA | 60:40 | |
| 5 | PVP | Ceraphyl 50 | LA | 60:10:30 | |
| 6 | PVP | — | Oleic acid | 50:50 | 30,000 |
| 7 | PVP | — | Octanoic acid | 55:45 | 7,000 |
| 8 | PVP | Polysorbate 80 | — | 50:50 | |
| 9 | PVP | ― | PEG 400 | 50:50 | |
| 10 | PVP | Caster oil | — | 50:50 | |
| 11 | | Pluronic 105 | ― | 100 | 1,000,000 |
| 12 | PVP | | glyerin | 50:50 | 5,000 |
| 13 | PVP | | BB/BA=9/1 | 51:49 | |

| | | | | | |
|---|---|---|---|---|---|
| GML = glycerol monolaurate LL = lauryl lactate PVP = polyvinylpyrrolidine LA = lauryl alcohol PEG = polyethyleneglycol 400 | | | | | |

### Example 2: Preparation of Human Growth Hormone Particles

Individual solutions of human growth hormone (hGH), sucrose, and mothionine were prepared in 5.0 mM Tris buffer, pH 7.4. The three solutions were then mixed to obtain an hCTH/sucrose/methionine/Tris solution having weight ratios of hGH/sucrose/methionine/Tris of 100/200/100/9.1, respectively. The solution was centrifuged at 3500 rpm for 15 min at 4°C, and the supernatant was spray-dried using the following conditions:

| | |
|---|---|
| Atomizing air pressure | 0.2 MPa |
| Air flow | 0.43 m³/min |
| Inlet temperature | 121-122°C |
| Outlet temperature | 87°C |
| Spray dryer exhaust humidity | 4-5 % |
| Spray dryer exhaust temperature | 70°C |
| Solution flow rate | 4 ml/min |

hGH particles were obtained, the majority of which were in the size range of 2 to 20 microns.

### Example 3: Preparation of hGH/Zn complexes

A 40 mg/mL hGH solution and a 27.2 mM zinc acetate solution were prepared in 5mM TRIS buffer, pH 7.0. Equal parts of the hGH and zinc acetate solutions were mixed to yield a zinc/hGH solution having a molar ratio of zinc to hGH of 15: 1. The zinc/hGH solution was allowed to complex for approximately one hour at 4°C. hGH/Zn particles were prepared by spray drying using the following conditions:

| | |
|---|---|
| Atomizing air pressure | 0.2 MPa |
| Air flow | 0.43 m³/min |
| Inlet temperature | 121-122°C |
| Outlet temperature | 87°C |
| Spray dryer exhaust humidity | 4 - 5 % |
| Spray dryer exhaust temperature | 70°C |
| Solution flow rate | 4 ml/min |

hGH/Zn particles were obtained, the majority of which were in the size range of 2 to 20 microns.

### Example 4: Protein particle solubility tests

The solubility of hGH/Zn particles in various media was determined. hGH/Zn particles (4 - 8 mg) were added to a 1.5 ml Ependorph tube that contained 1.0 ml of deionized water (DI water), sodium phosphate buffer (50 mM, pH 7.4, containing 150 mM Nacl) (PBS), or PBS with 20 nS4 ethylenediaminetetraacetic acid (EDTA). The Ependorph tube was slowly rotated end-over-end at room temperature for 30 minutes, and was then centrifuged at 10,000 rpm for 1 minute. The concentration of hGH in the supernatant was determined by measuring UV absorbance. The solubility of hGH/Zn was expressed as the fraction of dissolved hGH, and is shown in Table 2. While essentially insoluble in DI water, the hGH/Zn complex was soluble in PBS. Table 2 also shows that formation of the hGH/Zn complex is fully reversible by the chelating agent EDTA.

**Table 2**

| **Medium** | **Average solubility (%)** | **SD** |
|---|---|---|
| DI water | 0.013% | 0.020% |
| PBS | 95.03% | 1.85% |
| DI water + EDTA | 95.21% | 4.90% |

### Data are the average of triplicates.

### Example 5: Suspension preparation

hGH and Zn/hGH particles prepared as described in Examples 2 and 3, respectively, were loaded into a non-aqueous single phase viscous vehicle (Formulation 13 prepared as described in Example 1). The compositions of the formulations are shown in Table 3. The protein particles and the non-aqueous single phase viscous vehicle were added to a glass vessel in a nitrogen filled glove box. The mixture was manually stirred with a spatula to wet the protein particles completely. The mixture was further stirred at 65°C with a spatula attached to an overhead mixer until a homogeneous suspension was achieved.

**Table 3**

| **Formulation** | **Type of protein particle** | **Particle loading (%)** | **Formulation 13 (%)** |
|---|---|---|---|
| 14 | hGH | 9.30 | 96.50 |
| 15 | hGH/Zn | 2.66 | 97.74 |

### Example 6: hGH stability studies

The stability of hGH particles in non-aqueous vehicles was determined according to the following procedures. hGH particles suspended in non-aqueous vehicles were spiked with either DI water or 50 mM sodium phosphate buffer (PBS), pH 7.4 containing 150 mM NaCl (Table 4). The suspensions were stirred gently until the DI water or PBS was mixed homogenously with the non-aqueous vehicle. The suspensions were then incubated at 37 °C. The hGH monomer content and the total protein recovery (the fraction of water soluble hGH) were determined using size exclusion chromatography (SEC).

**Table 4**

| **Formulation** | **Non-aqueous vehicle** | **Type of aqueous solution** | **Aqueous solution content (%)** |
|---|---|---|---|
| 16 | 14 | N/A | 0 |
| 17 | 14 | DI Water | 5 |
| 18 | 14 | DI Water | 20 |
| 19 | 14 | PBS | 5 |
| 20 | 14 | PBS | 20 |
| 21 | 15 | N/A | 0 |
| 22 | 15 | DI Water | 5 |
| 23 | 15 | DI Water | 20 |
| 24 | 15 | PBS | 5 |
| 25 | 15 | PBS | 20 |

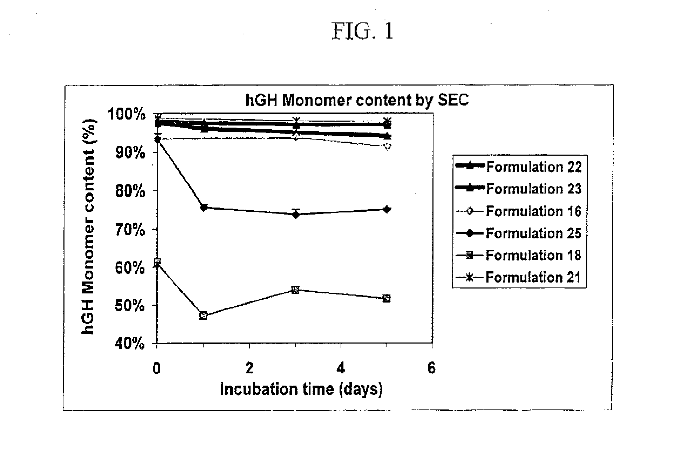

Figure 1 shows the hGH monomer content as a function of incubation time after adding aqueous media as indicated in Table 4. Comparing Formulation 23 with Formulation 18, it is apparent that the hGH/Zn complex was more stable than uncomplexed hGH. The hGH/Zn complex exhibited a significant decrease in monomer content in the vehicle containing 20% PBS, however, due to decomplexation of the hGH/Zn in PBS, resulting in soluble hGH. As shown in Table 2 of Example 4, the hGH/Zn complex is insoluble in DI water but soluble in PBS.

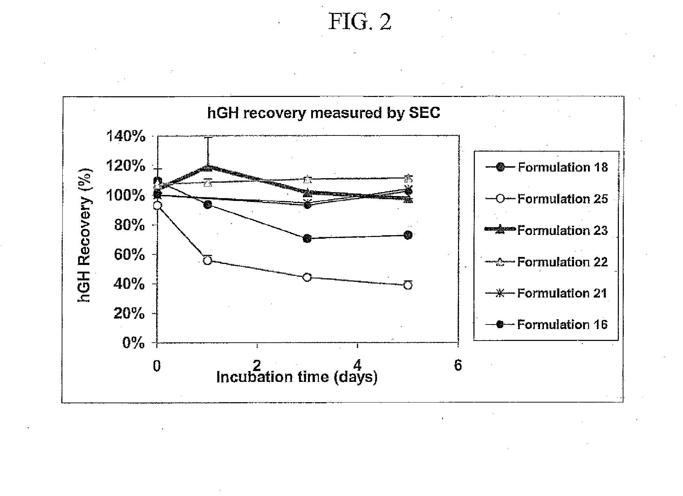

Figure 2 shows that the insoluble hGH/Zn complex is resistant to irreversible aggregation. Comparing Formulation 23 with Formulation 18, it is apparent that the hGH/Zn complex results in a significantly higher hGH recovery, or the fraction of soluble hGH, than uncomplexed hGH. Significant aggregation of the hGH/Zn complex occurred in the non-aqueous vehicle that contained 20 % PBS, however. *In vivo* delivery of these formulations would likely correspond to significantly shorter contact times of the formulation with aqueous media than the incubation times shown in Figure 2. As shown in Table 2 of Example 4, the hGH/Zn complex is soluble in PBS while insoluble in DI water.

### Example 7: Preparation of omega-interferon/zinc complexes

Solutions of either 5 mM acetate buffer at pH 5.5 or 5 mM tris buffer at pH 7.5, 8.3 or 9.6 were prepared. Zinc acetate was added to the buffer solution to create a solution of 5 mM zinc acetate. Omega-IFN was added to a separate vial of the same buffer to create a solution of 1 mg/mL omega-IFN. The solution containing zinc was added to the omega-IFN solution in an appropriate quantity to create a mixture with a zinc:omega-IFN molar ratio in the range of 1:1 to 50:1.

### Example 8: Determination of the percentage of soluble omega-interferon

To determine the fraction of soluble omega-IFN, zinc and omega-IFN were first combined as described above. The resulting mixture was observed visually, centrifuged, and the quantity of soluble omega-IFN in the clear supernatant was measured. By comparing the total quantity of omega-IFN in the mixture, and the quantity of omega-IFN in the supernatant, the percentage of soluble ornega-TFN was determine. The data obtained (Table 5, below) indicate that the fraction of soluble omega-IFN varies with the pH and the ratio of zinc to omega-IFN.

**Table 5**

| | **pH (buffer type, 5 mM)** | | | |
|---|---|---|---|---|
| **Molar ratio Zn:omega-IFN** | **5.6 (acetate)** | **7.5 (TRIS)** | **8.3 (TRIS)** | **9.6 (TRIS)** |
| 0:1 | Clear | Clear | Clear | Clear |
| 2:1 | Clear | Clear | Clear | Cloudy, not measured |
| 10:1 | 89% | 78% | 35% | 10% |
| 50:1 | 72% | 71% | 42% | 67% |

### Example 9: Microcalorimetry measurements of zinc/omega-IFN complexes

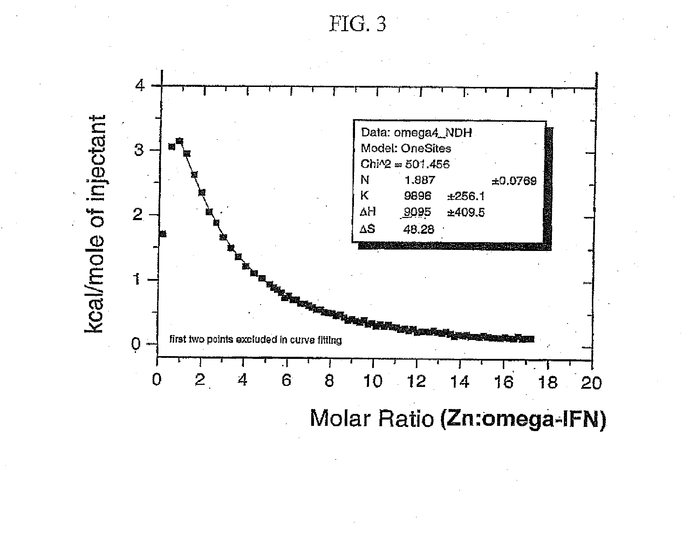

Microcalorimetry experiments were performed on mixtures of zinc and omega-IFN at pH 5.5 in 5 mM acetate buffer. Known quantities of 5 mM zinc acetate in 5 mM acetate buffer were slowly added to a 1 mg/mL omega-IFN solution in 5 mM acetate buffer that was being stirred at a constant temperature. Following each injection of zinc solution into the omega-IFN solution, the heat evolved was recorded in kcal per mole of injectant. The data obtained (Figure 3) indicate that heat was absorbed as the zinc was added to the omega-IFN solution, indicating that complexation occurred between the zinc ion and the omega-IFN molecule.

The entire disclosure of each patent, patent application, and publication cited or described in this document is hereby incorporated herein by reference.

### ADDITIONAL ASPECTS OF THE PRESENT INVENTION

1. A formulation comprising a complex of a metal ion and a polypeptide suspended in a non-aqueous, biocompatible suspension vehicle.
2. The formulation of 1 wherein the polypeptide/metal ion complex is insoluble in aqueous media.
3. The formulation of 1 wherein the metal ion is a multivalent metal ion.
4. The formulation of 3 wherein the multivalent metal ion is zinc, magnesium, calcium, nickel, or copper.
5. The formulation of 1 wherein the polypeptide is human growth hormone.
6. The formulation of 1 wherein the molar ratio of the metal ion to the polypeptide in the metal ion/polypeptide complex is from 1:1 to 100:1.
7. The formulation of 1 wherein the non-aqueous, biocompatible suspension vehicle comprises at least one of a polymer, a solvent, and a surfactant.
8. The formulation of 7 wherein the polymer is a polyester, a pyrrolidone, an ester or ether of an unsaturated alcohol, or a polyoxyethylenepolyoxypropylene block copolymer.
9. The formulation of 8 wherein the polyester is polylactic acid or polylacticpolyglycolic acid, the pyrrolidone is polyvinylpyrrolidone, the ester or ether of an unsaturated alcohol is vinyl acetate, and the polyoxyethylenepolyoxypropylene block copolymer is Pluronic 105.
10. The formulation of 7 wherein the solvent is a carboxylic acid ester, a polyhydric alcohols, a polymer of a polyhydric alcohol, a fatty acid, an oil, an ester of a polyhydric alcohol, propylene carbonate, benzyl benzoate, lauryl alcohol, or benzyl alcohol.
11. The formulation of 10 wherein the carboxylic acid ester is lauryl lactate, the polyhydric alcohol is glycerin, the polymer of a polyhydric alcohol is polyethylene glycol, the fatty acid is oleic acid or octanoic acid, the oil is castor oil, and the ester of a polyhydric alcohol is triacetic acetate.
12. The formulation of 7 wherein the surfactant is an ester of a polyhydric alcohol, ethoxylated castor oil, a polysorbate, an ester or ether of a saturated alcohol, or a polyoxyethylenepolyoxypropylene block copolymer.
13. The formulation of 12 wherein the ester of a polyhydric alcohol is glycerol monolaurate, the ester or ether of a saturated alcohol is myristyl lactate, and the polyoxyethylenepolyoxypropylene block copolymer is Pluronic.
14. A method for improving the stability upon exposure to aqueous media of a polypeptide suspended in a non-aqueous biocompatible suspension vehicle comprising forming a complex of the polypeptide and a metal ion.
15. The method of 14 wherein the polypeptide/metal ion complex is insoluble in aqueous media.
16. The method of 14 wherein the metal ion is a multivalent metal ion.
17. The method of 16 wherein the multivalent metal ion is zinc, magnesium, calcium, nickel, or copper.
18. The method of 14 wherein the polypeptide is human growth hormone.
19. The method of 14 wherein the molar ratio of the metal ion to the polypeptide in the metal ion/polypeptide complex is from 1:1 to 100:1.
20. The method of 14 wherein the suspension vehicle comprises at least one of a solvent, a polymer, and a surfactant.
21. The method of 20 wherein the polymer is a polyester, a pyrrolidone, an ester or ether of an unsaturated alcohol, or a polyoxyathylenepolyoxypropylene block copolymer.
22. The method of 21 wherein the polyester is polylactic acid or polylacticpolyglycolic acid, the pyrrolidone is polyvinylpyrrolidone, the ester or ether of an unsaturated alcohol is vinyl acetate, and the polyoxyethylenepolyoxypropylene block copolymer is Pluronic 105.
23. The method of 20 wherein the solvent is a carboxylic acid ester, a polyhydric alcohol, a polymer of a polyhydric alcohol, a fatty acid, an oil, an ester of a polyhydric alcohol, propylene carbonate, benzyl benzoate, lauryl alcohol, or benzyl alcohol.
24. The method of 23 wherein the carboxylic acid ester is lauryl lactate, the polyhydric alcohol is glycerin, the polymer of a polyhydric alcohol is polyethylene glycol, the fatty acid is oleic acid or octanoic acid, the oil is castor oil, and the ester of a polyhydric alcohol is triacetic acetate.
25. The method of 20 wherein the surfactant is an ester of a polyhydric alcohol, ethoxylated castor oil, a polysorbate, an ester or ether of a saturated alcohol, or a polyoxyethylenepolyoxypropylene block copolymer.
26. The method of 25 wherein the ester of a polyhydric alcohol is glycerol monolaurate, the ester or ether of a saturated alcohol is myristyl lactate, and the polyoxyethylenepolyoxypropylene block copolymer is Pluronic.

## Claims

1. A formulation comprising a complex of a metal ion and a polypeptide suspended in a non-aqueous, biocompatible suspension vehicle.

2. The formulation of claim 1 wherein the polypeptide/metal ion complex is insoluble in aqueous media.

3. The formulation of claim 1 wherein the metal ion is a multivalent metal ion.

4. The formulation of claim 3 wherein the multivalent metal ion is zinc, magnesium, calcium, nickel, or copper.

5. The formulation of claim 1 wherein the polypeptide is human growth hormone.

6. The formulation of claim 1 wherein the molar ratio of the metal ion to the polypeptide in the metal ion/polypeptide complex is from 1:1 to 100: 1.

7. The formulation of claim 1 wherein the non-aqueous, biocompatible suspension vehicle comprises at least one of a polymer, a solvent, and a surfactant.

8. The formulation of claim 7 wherein the polymer is a polyester, a pyrrolidone, an ester or ether of an unsaturated alcohol, or a polyoxyethylenepolyoxypropylene block copolymer.

9. The formulation of claim 8 wherein the polyester is polylactic acid or polylacticpolyglycolic acid, the pyrrolidone is polyvinylpyrrolidone, the ester or ether of an unsaturated alcohol is vinyl acetate, and the polyoxyethylenepolyoxypropylene block copolymer is Pluronic 105.

10. The formulation of claim 7 wherein the solvent is a carboxylic acid ester, a polyhydric alcohol, a polymer of a polyhydric alcohol, a fatty acid, an oil, an ester of a polyhydric alcohol, propylene carbonate, benzyl benzoate, lauryl alcohol, or benzyl alcohol.

11. The formulation of claim 10 wherein the carboxylic acid ester is lauryl lactate, the polyhydric alcohol is glycerin, the polymer of a polyhydric alcohol is polyethylene glycol, the fatty acid is oleic acid or octanoic acid, the oil is castor oil, and the ester of a polyhydric alcohol is triacetic acetate.

12. The formulation of claim 7 wherein the surfactant is an ester of a polyhydric alcohol, ethoxylated castor oil, a polysorbate, an ester or ether of a saturated alcohol, or a polyoxyethylenepolyoxypropylene block copolymer.

13. The formulation of claim 12 wherein the ester of a polyhydric alcohol is glycerol monolaurate, the ester or ether of a saturated alcohol is myristyl lactate, and the polyoxyethylenepolyoxypropylene block copolymer is Pluronic.

14. A method for improving the stability upon exposure to aqueous media of a polypeptide suspended in a non-aqueous biocompatible suspension vehicle comprising forming a complex of the polypeptide and a metal ion.

15. The method of claim 14, wherein the complex is as defined in claim 2 or 6, or the metal ion is as defined in claim 3 or 4, or the polypeptide is as defined in claim 5, or the vehicle is as defined in any one of claims 7 to 13.
